# EUROPEAN PATENT APPLICATION

(11) **EP 3 156 401 A1**
(43) Date of publication of application: **19.04.2017**
(21) Application number: 16192112.7
(22) Date of filing: 03.10.2016
(51) Int. Cl.: C07D 303/23, C09D 1/00

(54) **TRISTYRYLPHENOL MONOGYCIDYL ETHER**

(30) Priority: 15.10.2015 US 201562241911 P
(71) Applicant: Dow Global Technologies LLC, Midland, MI 48674 (US); Rohm and Haas Company, Philadelphia, PA 19106 (US)
(72) Inventor: BRENNAN, David J., Midland, MI 48667 (US); CARDOEN, Gregoire, Collegeville, PA 19426 (US); HEFNER, Jr., Robert E., Freeport, TX 77541 (US); VAN DYK, Antony K., Collegeville, PA 19426 (US)
(74) Representative: Mauro, Marina Eliana

(57) **Abstract**

The present invention relates to a compound having the following structure: 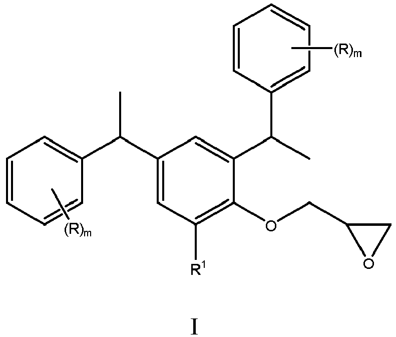 where R, R¹, and m are as defined herein. The compound is useful as a precursor to a surfactant with a low hydrophilic-lipophylic balance (HLB), which can be used to increase open time for coatings.

## Description

### Background of the Invention

The present invention relates to a tristyrylphenol monoglycidyl ether and its preparation. This compound is useful as a precursor for a surfactant with a low hydrophilic lipophilic balance (HLB); such a surfactant can be used an additive to improve open time in a coatings formulation.

Government regulations and market movement continually drive toward zero volatile organic compounds (VOC) for coating formulations. Consequently, waterborne formulations that are free of volatile solvents and coalescents have become increasingly popular in the industry. Nevertheless, paint properties have been compromised due to this sea change; among them is open time, which is the period of time during which a freshly applied paint film can be reworked without leaving brush marks. In a solvent-borne system, open time is about 30 to 45 min; in a typical waterborne formulation, open time is in the order of 3 to 5 min. Accordingly, there is a need in the art to find an additive for waterborne formulations that increases open time over currently available additives without degrading other properties of the final coating, such as film adhesive and cohesive strength, hardness, block resistance, early blister resistance, scrub and wash resistance, stain resistance, and mar resistance.

### Summary of the Invention

The present invention addresses a need in the art by providing a compound represented by the following structure: 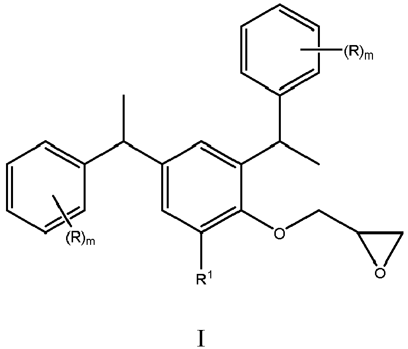 where each R is independently F, Cl, Br, CN, C₁-C₆-alkyl, or C₁-C₆-alkoxy; R¹ is H or 1-phenylethyl; and each m is independently 0, 1, 2, or 3. The compound of the present invention is useful as a precursor to a surfactant with a low hydrophilic-lipophylic balance (HLB).

### Detailed Description of the Invention

The present invention is a compound represented by the following structure: 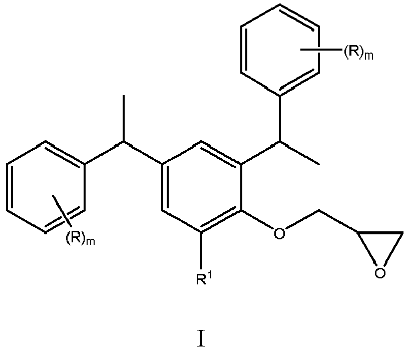 where each R is independently F, Cl, Br, CN, C₁-C₆-alkyl, or C₁-C₆-alkoxy; R¹ is H or 1-phenylethyl; and each m is independently 0, 1,2, or 3. R is preferably C₁-C₄-alkyl, more preferably methyl or ethyl, and most preferably methyl; R¹ is preferably 1-phenylethyl; and m is preferably 0, 1, or 2, more preferably 0 or 1, and most preferably 0.

The compound of Formula I can be prepared by contacting under reactive conditions an epihalohydrin with a tristyryl monophenol of Formula II: 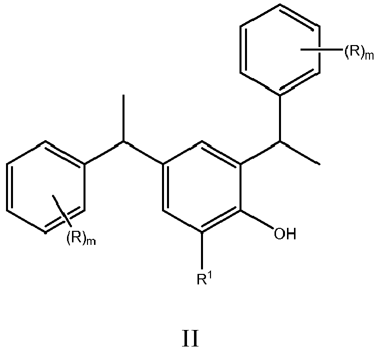 where R, R¹, and m are as previously defined.

For example, the compound of Formula I can be prepared by contacting an epihalohydrin with the compound of Formula II in the presence of a base such as an alkali metal or alkaline earth metal hydroxide, carbonate, or bicarbonate, or an alkali metal hydride. Examples of suitable bases include NaOH, KOH, Na₂CO₃, K₂CO₃, NaHCO₃, KHCO₃, NaH, and KH, with aqueous NaOH being preferred. A preferred epihalohydrin is epichlorohydrin.

The process may be carried out in the presence of a suitable solvent such as toluene, methylisobutyl ketone, methylene chloride, or isopropanol. Alternatively, the reaction may be conducted without any ancillary solvent, wherein epihalohydrin plays the role of both reagent and solvent. In any case, the epihalohydrin is advantageously used in stoichiometric excess with respect to the compound of Formula II.

The process is typically performed at or around atmospheric pressure, at a temperature preferably in the range of from 25 °C to 70 °C, and for a time to achieve the desired yield.

Recovery and purification of the desired product can be carried out by a variety of methods well known in the art; where epichlorohydrin is used as a solvent, vacuum distillation is advantageously used for removal and recycling.

The compound of Formula I can be used as a precursor to a surfactant that is useful in improving open time for coatings. For example, the compound can be reacted with a hydrophilic amine or alcohol under oxirane ring opening conditions to form the surfactant or yet another precursor to the surfactant. Examples of suitable hydrophilic amines include N-methylethanol amine, monoethanol amine, and diethanol amine, which can be reacted in the presence of a base with the compound of Formula I to make compounds of Formulas III, IV, and V:

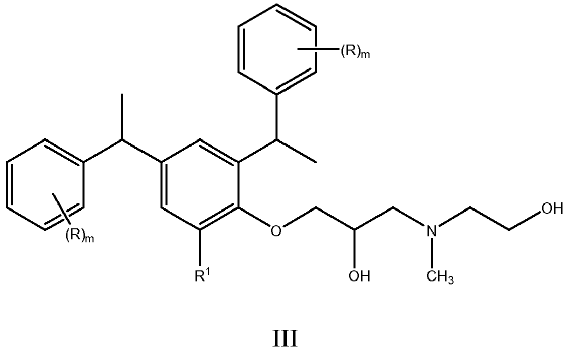

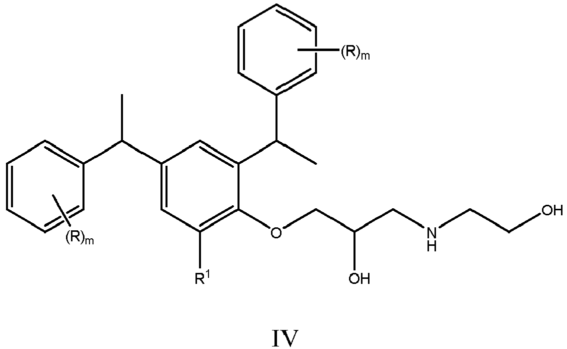

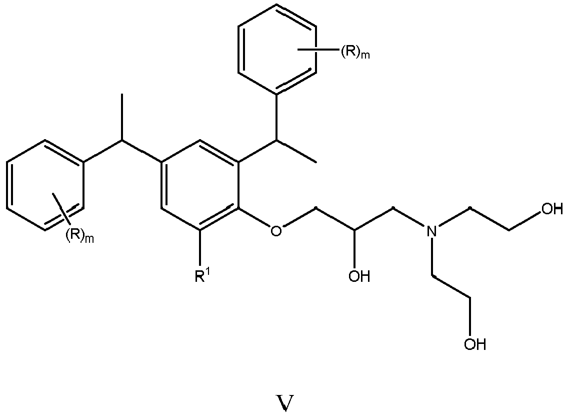

While not being bound to theory, it is believed that the bulky hydrophobic tristyryl group has a strong affinity to the latex particle surface and forms a protective layer around the colloid while the hydrophilic portion creates steric repulsion between particles. These features result in a delay of latex particle coalescence thereby increasing open time.

### Examples

### Example 1 - Preparation of Tristyrylphenol Monoglycidyl Ether

A 2-L, 3-neck round bottom reactor was charged with tristyrylphenol (the compound of Formula II where m = 0, 200.0 g, obtained from Saltigo GmbH, Leverkusen, DE, 66% 1,3,5-tristyrylphenol, 26% 2,6-distyryl phenol) and epichlorohydrin (455.4 g). Isopropanol (245.2 g) was then added with stirring, followed by the addition of deionized (DI) water (39.6 g). The contents of the reactor were heated to 51 °C, whereupon aqueous NaOH (17.7 g in 70.9 g DI water) was added dropwise over 20 min. The mixture was heated and stirred for an additional 20 min, after which time the contents were allowed to settle for 4 min to form a biphasic mixture. The aqueous layer was removed from the reactor leaving a clear organic material. The contents were heated to 50 °C with stirring for 4 min, at which time a second portion of aqueous NaOH (7.9 g in 31.5 g water) was added dropwise over 15 min. The reactants were stirred and heated for an additional 20 min, after which time the reactor contents were allowed to settle to form a biphasic mixture. The aqueous layer was removed leaving a clear light yellow colored organic layer.

The contents were once again heated to 50 °C with stirring for 1 min, after which time a third portion of aqueous NaOH (2.0 g in 7.9 g DI water) was added dropwise over 4 min. The reactants were stirred and heated for an additional 16 min, after which time the contents of the reactor were transferred to a separatory funnel and allowed to settle. The aqueous layer was removed and the organic portion washed three times with DI water. For the third washing the biphasic mixture was allowed to settle for 45 min. The resultant organic layer was dried over Na₂SO₄ supported in a fritted glass funnel on a side arm flask, then vacuum filtered. Solvent was removed *in vacuo* to give a transparent light yellow colored viscous liquid (215.19 g), which was found to be a mixture of the monoglycidyl ether of tristyrylphenol and the monoglycidyl ether of distyryl phenol, confirmed by epoxide titration and gas chromatographic analysis.

## Claims

1. A compound represented by the following structure: 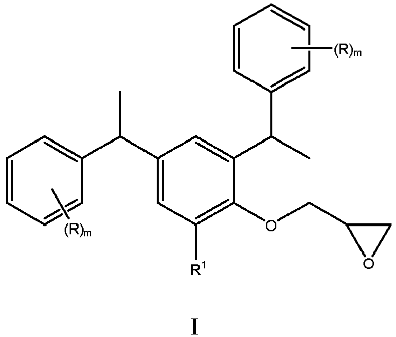 where each R is independently F, Cl, Br, CN, C₁-C₆-alkyl, or C₁-C₆-alkoxy; R¹ is H or 1-phenylethyl; and each m is independently 0, 1, 2, or 3.

2. The compound of Claim 1, wherein each R is methyl, ethyl, methoxy, or ethoxy; and m is 1 or 2.

3. The compound of Claim 1, wherein m is 0.

4. The compound of Claim 3, wherein R¹ is 1-phenethyl.

5. The compound of Claim 1, wherein R¹ is H.
